# FASCICULE DE BREVET EUROPEEN

(11) **EP 0 752 863 B1**
(45) Date de publication et mention de la délivrance du brevet: **04.12.2002**
(21) Numéro de dépôt: 95915223.2
(22) Date de dépôt: 29.03.1995
(51) Int. Cl.: A61K 31/715, A61P 1/04

(54) **UTILISATION DE BIOPOLYMERES POUR LE TRAITEMENT DE LESIONS DU TRACTUS DIGESTIF**
VERWENDUNG VON BIOPOLYMEREN ZUR BEHANDLUNG VON VERLETZUNGEN DES VERDAUUNGSTRAKTES
USE OF BIOPOLYMERS FOR DIGESTIVE TRACT INJURIES TREATMENT

(30) Priorité: 30.03.1994 FR 9403804
(43) Date de publication de la demande: 15.01.1997
(73) Titulaire: Valbiofrance, 75006 Paris (FR)
(72) Inventeur: BARRITAULT, Denis, F-75001 Paris (FR); CARUELLE, Jean-Pierre, F-94100 Saint-Maur (FR); MEDDAHI, Anne, F-94000 Créteil (FR)
(74) Mandataire: Michelet, Alain
(86) Numéro de dépôt international: FR9500399
(87) Numéro de publication internationale: WO95026737

(56) Documents cités:
- FR-A- 2 461 724
- FR-A- 2 644 066
- MINERVA DIETOL. GASTROENTEROL., vol. 31, no. 2, 1985 pages 311-315, A. SAGGIORO ET AL. 'Treatment of hemmorhoidal syndrome with mesoglycan sulfate.'
- BOLL. CHIM. FARM., vol. 119, no. 8, 1980 pages 487-498, G. CORBELLI ET AL. 'Evaluation of the stability of vessel, a glycosaminoglycan sulfate of extractive origin, and heparin in human digestive juices.'

## Description

La présente invention a pour objet l'utilisation de polymères ou de biopolymères pour la préparation d'un médicament pour le traitement de lésions de toutes origines affectant le tractus digestif de la cavité buccale à l'extrémité anale en médecine humaine ou vétérinaire .

Elle est en outre relative a une composition contenant ces polymères et destinée à un tel traitement.

La synthèse des polymères CMDBS (Carboxy Méthyl Dextrane Benzylamine Sulfonate) a été décrite dans le brevet FR 2 461 724 ainsi que dans le brevet US 4 740 594. Certains de ces polymères miment l'héparine et peuvent être utilisés en tant que produits de remplacement de l'héparine du plasma, grâce à leurs propriétés anticoagulante et anticomplément.

Parmi l'ensemble des polymères CMDBS, certains miment une autre propriété de l'héparine qui consiste en une stabilisation, protection et potentialisation de l'activité biologique in vitro des facteurs de croissance de la famille FGF (Tardieu et coll , Journal of Cellular Physiology,1992,150 pages 194 à 203).

Le brevet FR 2 644.066 décrit l'utilisation de certains CMDBS associés aux FGF pour la cicatrisation de la peau et de la cornée. Des expériences ont été réalisées en provoquant une blessure cutanée à l'aide d'un emporte pièce de 6 mm de diamètre chez le rat. Dans cet exemple, le CMDBS associé au FGF 2 permet d'obtenir un effet net sur la vitesse et la qualité de la réparation de la peau.

Un autre biopolymère, le dextrane sulfate a également été proposé en association avec des FGF, comme stabilisateur et protecteur, dans le. brevet Japonais N°13890. Le dextrane sulfate est par ailleurs largement utilisé dans des pommades ou crèmes cicatrisantes de la peau ainsi que dans des compositions de collyre, mais n'a aucun effet reporté à la connaissance du demandeur sur la cicatrisation et ou la régénération de lésions du tractus digestif.

Un autre agent, le sucrose sulfate ester et son sel d'aluminium le sucralfate sont des produits décrits et utilisés comme agents de traitement des ulcères et lésions du tractus digestifs (Brevet US N°3,432,489) et dans différentes associations et compositions pharmaceutiques décrites dans une série de brevets (US N°s4975281, 4885281, 5013557, 5164379, 5196405, 5240710 et DK N°102488 et N°505588).

Les tissus du tractus digestif sont particulièrement riches en facteurs de croissance et plusieurs auteurs ont décrit la présence et/ou l'action des FGF et TGF bêta dans/sur les cellules entérocytes ou l'action cicatrisante de ces facteurs dans des lésions des tissus du tractus digestif ainsi que la présence ou l'action d'autres facteurs de croissance présentant une affinité pour l'héparine ou l'héparane tels les PDGF AB ou BB ou l'Hépatocyte Growth Factor (LEMOINE NR; LEUNG HY; GULLICK WJ: Growth Factors in the Gastrointestinal tract; Gut 1992, 33, pp. 1297 à 1300; Di GULIETTA A, HERVADA T; NARDY RV; LESH CA: Effect of platelet derived growth factor BB on indomethacin-induced gastric lésions in rats; Scand J. Gastroenterol 1992, 27, pp. 673 à 676; TAJAGASGU M et coll. Hepatocyte growth factor induces mitogenic reaction to the rabbit gastric epithelial cells in primary culture; Biochem. and Biophys. Res. Comm. 1993, 191; 528-534; MUSTOE et coll. Differential acceleration of healing of surgical incisions in the rabbit gastrointestinal tract by platelet derived growth factor and transforming growth factor beta; Surgery 1990, 108 pp. 324 to 330; Katayama M; Kan M: Haprin-binding (fibroblast) growth factors are potential autocrine regulators of oesophageal epithelial cell proliferation, In Vitro Cell. Dev. Biol. 1991, 27, pp. 533 to 541).

Il ressort donc de l'analyse de l'état de la technique que des polymères ont déjà été utilisés en association avec des facteurs de croissance sur certaines lésions d'un type bien précis de tissu, le tissu cutané.

Du fait de l'imprévisibilité des effets thérapeutiques d'une molécule donnée, il n'était pas évident que ces polymères, seuls , et non associés à des facteurs de croissance, puissent avoir un effet sur d'autres tissus que ceux de la peau.

En effet, il est bien connu que les différents tissus du corps humain ou animal présentent des spécificités tant structurelles que fonctionnelles qui rendent impossible toute prédiction quant à l'effet de la molécule, connue pour son effet sur le tissu cutané, sur les tissus du tractus digestif.

Ceci est d'autant plus vrai que les tissus du tractus digestif et les tissus cutanés par exemple sont d'origines embryonnaires différentes.

De même, il est bien connu qu'il est impossible de prédire l'activité in vivo d'une molécule sur un tissu particulier à partir de résultats obtenus in vitro sur un modèle expérimental spécifique.

Tous les autres médicaments connus dans ce domaine, comme les anti-ulcéreux agissent en protégeant la muqueuse par des gels anti-acide, par des inhibitions des sécrétions d'acide gastrique ou par action anti-récepteur H2.

L'activité d'un mélange de glycosaminoglycanes, le mésoglycan, sur les hémorroïdes a été testée par Saggioro et al., (1985, Min. Diet. e. Gastr, 31, 311-315). Il ressort de cette publication que le mésoglycan est très efficace à l'encontre des hémorroïdes et permet d'éliminer les symptömes tout en améliorant l'aspect endoscopique.

Néanmoins cette publication est limitée à des polymères d'un type bien particulier, qui de plus présentent une composition d'origine animale mal définie et sujette à des variations.

De manière surprenante, il a été trouvé, selon l'invention, que certains polymères ont un effet très marqué sur la vitesse de cicatrisation et des lésions des tissus du tractus digestif ainsi que sur la qualité et la solidité de ces cicatrices.

Il a en outre été montré que des doses très faibles de ces polymères permettent d'obtenir des effets thérapeutiques.

La présente invention a pour objet une utilisation d'au moins un polymère ou d'un biopolymère, appelés HBGFPP, à l'exclusion du mésoglycan, protégeant spécifiquement les facteurs de croissance des familles des FGF et TGF bêta de la dégradation trypsique et n'inhibant pas de manière significative la coagulation, pour la fabrication d'un médicament pour le traitement des lésions du tractus digestif, et des tissus dérivés secondaires et primaires de l'endoderme et du mésoderme.

Un tel polymère présente particulièrement une activité anti-coagulante inférieure à 50 unités internationales par mg de polymère mesurée selon Maillet et al. (Mol. Immunol, 1988, 25, 915-923). Avantageusement il potentialise les FGF in vitro.

Préférentiellement, il n'active substantiellement pas le système du complément, c'est-à-dire qu'il possède une activité anti-complément supérieure à 0,5 µg pour le CH50 (selon Mauzac et al, Biomaterials, 6, 61-63,1985).

Selon la présente invention, on entend par polymères toutes substances naturelles, naturelles modifiées chimiquement ou totalement synthétiques répondant à la définition donnée ci-dessus.

Ainsi, il peut s'agir de :
- polymères obtenus à partir de dextranes mais modifiés par d'autres types de substitutions avec d'autres types de radicaux,
- polymères naturels autres que ceux dérivant de dextranes mais comportant des résidus osidiques (cellulose, chitine, fucanes, etc...),
- polymères obtenus par polymérisation de monomères de natures non osidiques (poly acide malique, poly acide oxaline, poly acide lactique, polystyrène, polyéthylène glycol) modifiés ou non.

Avantageusement, ledit polymère ou biopolymère est un polysaccharide qui peut être composé principalement de résidus glucose.

Il peut aussi comprendre des résidus glucosamine et/ou d'acide uronique, particulièrement sous la forme de dimère glucosamine-acide uronique.

Des polysaccharides particulièrement préférés sont des dextranes substitués, des glycosaminoglycanes, éventuellement associés à un lipide, un peptide ou un protide ou des sulfates de ces polymères.

Un tel polymère présente avantageusement un poids moléculaire d'au moins 10 kDa et préférentiellement d'environ 40 kDa.

Les polymères et/ou biopolymères peuvent être sélectionnés à partir de substances naturelles qui peuvent ensuite être éventuellement modifiées par additions de groupements chimiques appropriés, ou encore être obtenus entièrement par synthèse . Ces polymères naturels, semi synthétiques ou entièrement synthétiques sont ensuite sélectionnés sur la base de leurs capacités à interagir spécifiquement avec plusieurs facteurs de croissance notamment ceux de la famille des FGF et des TGF bêta. Ils sont également sélectionnés sur leurs capacité à protéger ce ou ces facteurs contre des dégradations protéolytiques. Ces polymères seront désignés sous le sigle générique de HBGFPP (heparin binding growth factor protectors and promotors}. Deux prototypes de ces polymères ou bio polymères sont donnés comme exemples ainsi que les procédés et critères de sélection de ces polymères:

Le premier exemple de HBGFPP appartient à la famille des CMDBS qui sont des produits connus, à savoir des dextranes biospécifiques fonctionnalisés, substitués par des résidus carboxyméthyle, benzylamide et benzylamine sulfonate. Ces polymères illustrent l'obtention de HBGFPP à partir de produits naturels (dextrans) subséquemment chimiquement substitués. Le deuxième exemple décrit la sélection de produits complètement naturels comme les proteoglycosaminoglycannes sulfates purifiés à partir d'extraits tissulaires.

Ces deux exemples illustrent les capacités de ces HBGFPP à d'interagir, à stabiliser, à protéger et à potentialiser les facteurs de croissances des familles FGF et TGF bêta et leur utilisation dans une composition pharmaceutique permettant la protection, la cicatrisation et/ ou la régénération de lésions de toutes origines affectant le tractus digestif de la cavité buccale à l'extrémité anale.

On entend, dans la présente demande, par traitement toute opération curative ou préventive effectuée- pour la prophylaxie et la cicatrisation de lésions du tractus digestif que ces lésions soient de types ulcéreux superficiels ou profonds et quelle qu'en soit l'origine, et/ou la cicatrisation des perforations et/ou des coupures ou découpes chirurgicales ainsi que des anastomoses effectuées sur les régions appropriées du tractus digestif.

Un médicament ou une composition pharmaceutique selon l'invention contient une quantité efficace de HBGFPP par exemple du CMDBS associé à un ou plusieurs véhicules compatibles et pharmaceutiquement acceptables. Il peut être également associé à des agents pharmaceutiques comme des agents antiinflammatoires, antibactériens, antifongiques ou des gels, emplâtres, ou pansements anti-acide, agents anti récepteurs H2 ou anti pompe à proton utilisés en traitement d'ulcères gastriques, ainsi qu'à des agents couramment utilisés en soins buccaux et dentaires ou pour le traitement des hémorroïdes.

Avantageusement, un tel médicament est conçu pour être directement absorbé par voie orale ou déposé sur la lésion si celle-ci est directement accessible notamment dans les lésions buccales ou rectales ou encore lors des interventions chirurgica.les en déposant ou imbibant les extrémités des tissus avant de les recoudre ou de les recoller ou d'effectuer des anastomoses. La dose unitaire est de 10 à 2500 µg de CMDBS ou de HBGFPP par ml.

Le véhicule peut être du sérum physiologique ou des tampons tels que le PBS, contenant NaCl 0.15 Molaire ou toute autre sorte de solution compatible et non irritante pour le tissu lésé. Des formulations permettant d'obtenir des solutions pâteuses ou en gel ou en aérosol selon les techniques courantes connues de l'homme de l'art peuvent être proposées selon le type et l'accessibilité de la lésion.

Ainsi, pour les lésions directement accessibles comme des aphtes buccaux, des lésions de la langue ou de la voûte palatale ou des gencives et de l'ensemble des tissus de support des dents ou de la gorge, la composition peut s'appliquer sous forme de solution, de suspension, d'aérosol, de poudre, de gel, d'onguent ou pommade, de pâte ou crème gélatineuse, de dentifrice de fixatif dentaire d'implant periodontal de pâte à mâcher, de tablettes effervescentes ou à sucer et peuvent également s'appliquer à l'aide d'une petite pipette ou d'une spatule ou d'un pinceau. L'effet de la composition décrite dans la présente invention est une guérison des lésions; que celles ci soient d'origine accidentelles comme des coupures ou des brûlures thermiques ou chimiques ou d'origines microbienne, fungiques ou virale (sous réserve pour ces origines que la cause de la lésion soit également traitée par une association avec des agents anti microbiens, antifongiques ou antiviraux) ou encore d'autres origines pas toujours identifiables ou connues ou enfin des lésions consécutives à une chimiothérapie ou radiothérapie

De même pour des lésions, maladies ou irritations anorectales, la composition objet de cette invention, offre un traitement qui réduit l'inflammation, l'irritation, les démangeaisons, le gonflement des tissus, et la souffrance causées par les maladies anorectales et conduit ces lésions vers la guérison en favorisant la réparation et la régénération des tissus. Les maladies anorectales concernent les régions périanales, le conduit anal et le rectum et incluent les hémorrhoïdes dont les causes peuvent être extrêmement variées comme la constipation, la diarrhée, la grossesse, les infections anales, les carcinomes rectales ainsi que d'autres désordres comme les fistules et fissures anales dont les causes peuvent être diverses parfois induites par-la chimio ou la radiothérapie. De même que pour les lésions des voies supérieures du tractus digestif, les lésions anorectales sont directement accessibles et la composition peut s'appliquer en solution, en suspension, sous forme d'aérosol, de poudre, de gel, d'onguent ou pommade, de pâte ou crème gélatineuse, de suppositoire et à l'aide d'une petite pipette ou d'une seringue dont l'embout est introduit dans le conduit anal ou d'une spatule ou d'un pinceau ou de tout autre moyen approprié.

La composition peut être associée avec d'autres compositions habituellement utilisées pour le traitement des maladies anorectales et dont la liste non exhaustive a été publiée par l'administration américaine fédérale des médicaments (FDA) dans une monographie N°45 35576 du 26 Mai 1980. Cette monographie décrit plus de 75 ingrédients classés par familles comme des anesthésiques locaux, des vasoconstricteurs, des protecteurs, des anti irritants, des agents astringents, des agents de cicatrisation, des antiseptiques, des keratolytiques et des anticholinergiques.

Les résultats obtenus par l'application de la présente composition sont sans commune mesure avec ceux obtenus avec les produits existants y compris les compositions à base de sucralfate comme elles sont décrites-dans le brevet US N°5 196 405 du 23 mars 1993 pour le traitement des hémorroïdes ou dans la demande US N°52407010 (DK102488 et DK505588). La dose de produit CMDBS ou HBGFP utilisée correspond selon la surface de la plaie à une fraction d'une solution de départ de 10 à 2500 ug par millilitre (ce qui pour une application locale sur des plaies courantes implique une dépose rarement supérieure à quelques centaines de microlitres de la solution) cette dose étant appliquée une ou deux fois par 24 heures. Les compositions utilisées de sucralfate sont de 0.01 à 5% (selon le brevet US N°5 196 405 ) soit au moins 10 fois supérieures à celles décrites dans la présente invention et les effets décrits dans tous les exemples de ce brevet US N'5 196405 sont obtenus à partir de compositions 50 milligrammes par millilitre.

Pour les lésions du tractus digestif non aisément accessibles par voie topique la voie d'administration de la composition est soit la voie orale soit la voie rectale. La présente composition à base de HBGFP est effective pour le traitement des lésions du pharynx, de l'estomac, des oesophages, du duodénum, de l'intestin grêle ou du colon. A titre d'exemples non exhaustif des lésions pouvant être traitées avec bénéfice par les compositions à base de HBGFP faisant l'objet de la présente invention on peut citer des lésions de la muqueuse de parties du tractus digestif provoquant des ulcères comme les ulcères acides gastriques ou duodénaux; les lésions induites par des médicaments, par des produits chimiques absorbés accidentellement ou par des traitements par des radiations, celles induites par le stress ou par des aliments ou d'origine chirurgicales ou traumatiques, virales ou celles associées à des inflammations comme les rectocolites hémorragiques, ou la maladie de Crohn.

L'activité stimulatrice de la réparation des tissus du tractus digestif peut être également envisagée pour d'autres tissus dérivés primaires ou secondaires de l'endoderme et du mésoderme comme les dérivés glandulaires, par exemple le foie, le pancréas, les glandes salivaires, l'adénohypophyse, ou comme les dérivés pharyngiens et respiratoires, par exemple les bronches, les poumons, les plèvres.

Les HBGPP peuvent donc être préconisés pour la réparation des lésions de toutes-origines affectant ces autres tissus dérivés primaires ou secondaires de l'endoderme et du mésoderme (lésion traumatique induite par la chirurgie ou par dees pathologies tumorales ou autres).

L'invention sera illustrée, sans être aucunement limitée par les exemples qui suivent, dans lesquels:

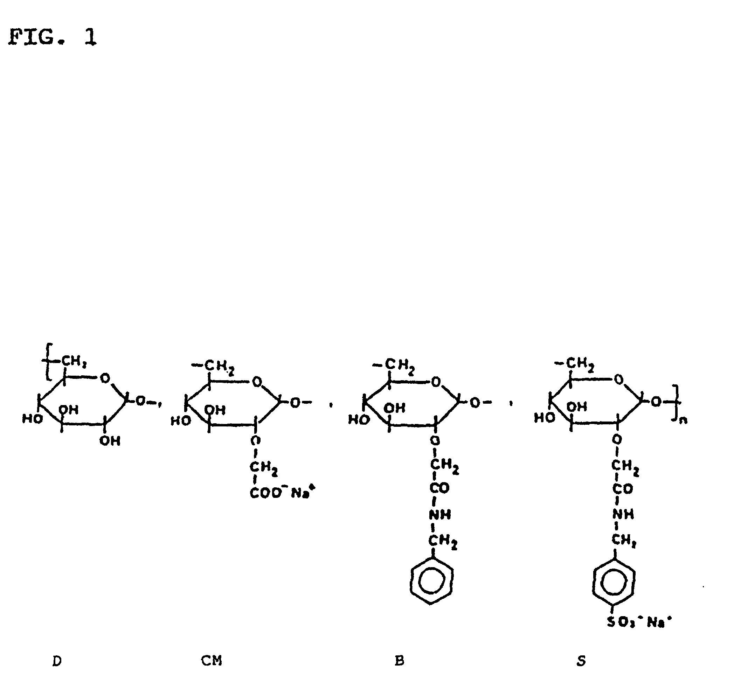

La figure 1 représente la formule du CMDBS.

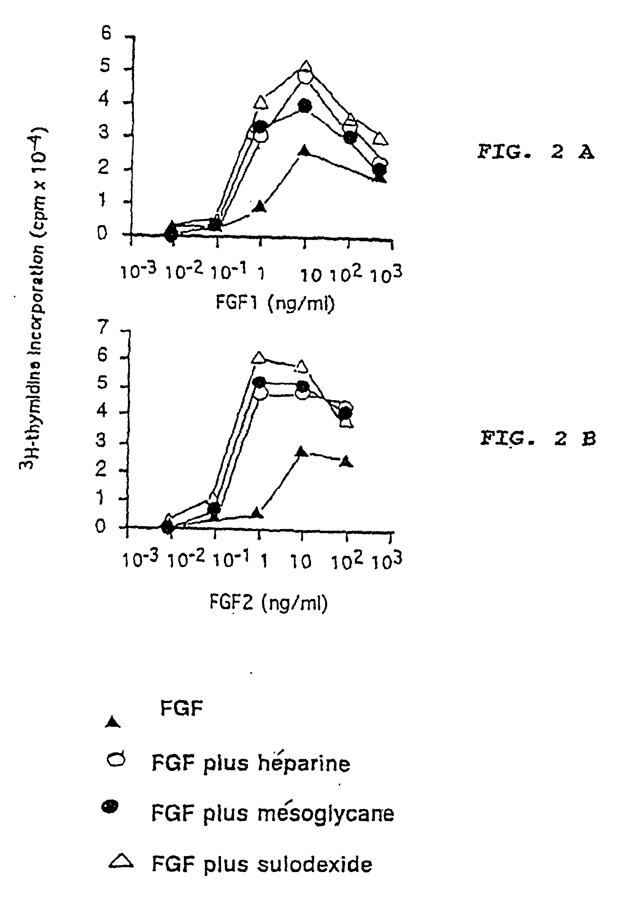

La figure 2 illustre la potentialisation de l'activité biologique des FGF1 (2a) et FGF2 (2b) par l'héparine, le mésoglycane et le sulodexide. La mesure de l'activité biologique est effectuée sur des cellules CCL39 par la mesure de l'augmentation de l'incorporation de thymidine tritiée en fonction de la dose de FGF1 et de FGF2 ajoutée seule ou en présence de 20 µg d'héparine, de 10 µg de mésoglycane, ou de 10 µg de sulodexide.

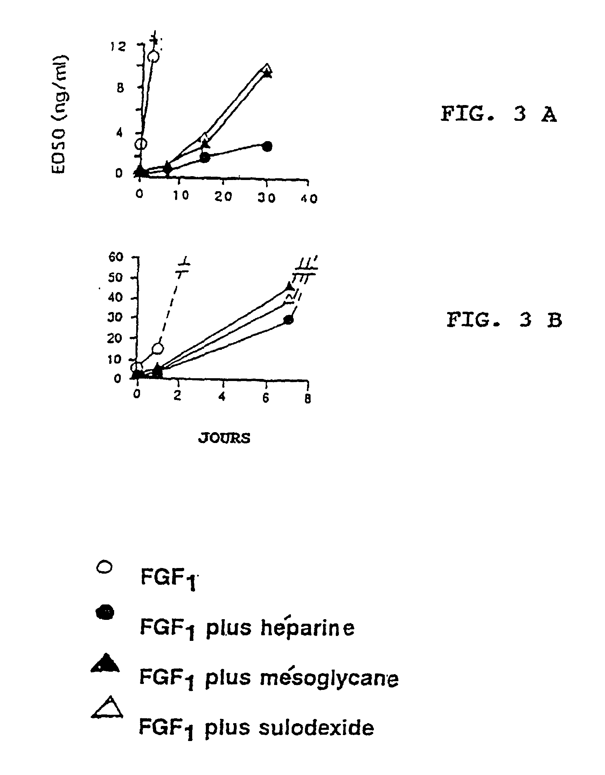

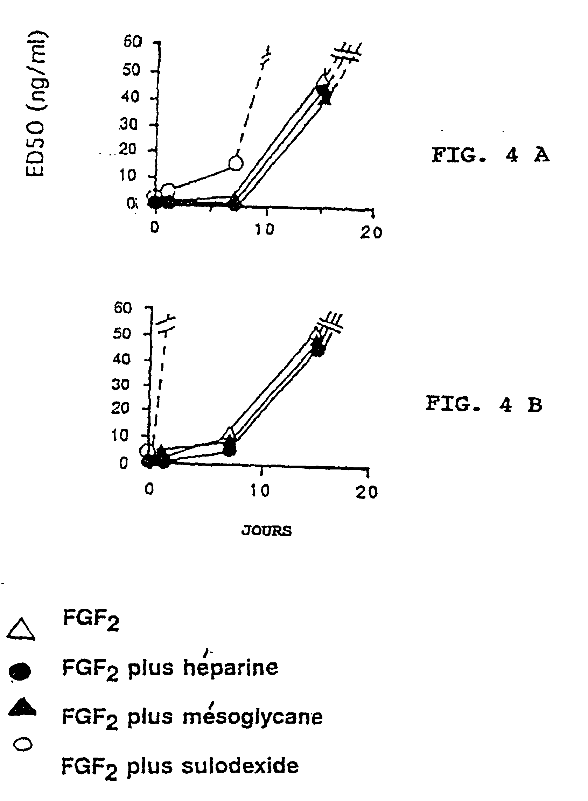

Les figures 3 et 4 illustrent l'effet protecteur de l'héparine, du mésoglycane et du sulodexide contre une dégradation thermique du FGF1(3) et FGF2 (4). Les échantillons de FGF sont incubés seuls ou en présence de 20 µg d'héparine, de 10 µg de mesoglycan ou de 10 µg de sulodexide à 20°C (a) et 37°C (b) pendant 1, 7, 15, 30 jours. La mesure de l'activité biologique présentée en abscisse correspond aux valeurs des unités de stimulation (ED50) de l'incorporation de thymidine tritiée dans des cellules CCL39.
La figure 5a illustre l'effet protecteur de l'héparine, du mésoglycan et du sulodexide centre une dégradation protéolytique du ¹²⁵I-FGT1. La digestion protéolytique a été effectuée à 37°C et les échantillons ont été séparés par électrophorèse sur gel de polyacrylamide à 18% . Les gels sont séchés et autoradiographiés. La première piste contient le ¹²⁵I-FGF1 seul, dans la deuxième (piste 2) le ¹²⁵I-FGF1 est incubé en présence de trypsine et d'héparine (piste 3), de mesoglycan (piste 4) ou de sulodexide (piste 5).

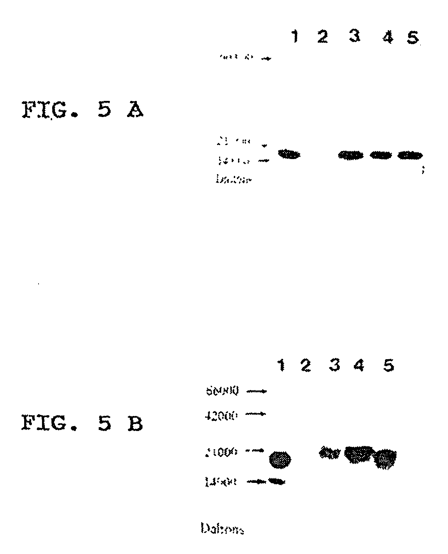

La figure 5b illustre l'effet protecteur de l'héparine, du mesoglycan et du sulodexide contre une dégradation protéolytique du ¹²⁵I-FGF2. La disposition des pistes est identique à celle présentée pour le ¹²⁵I-FGF1 en 5a.

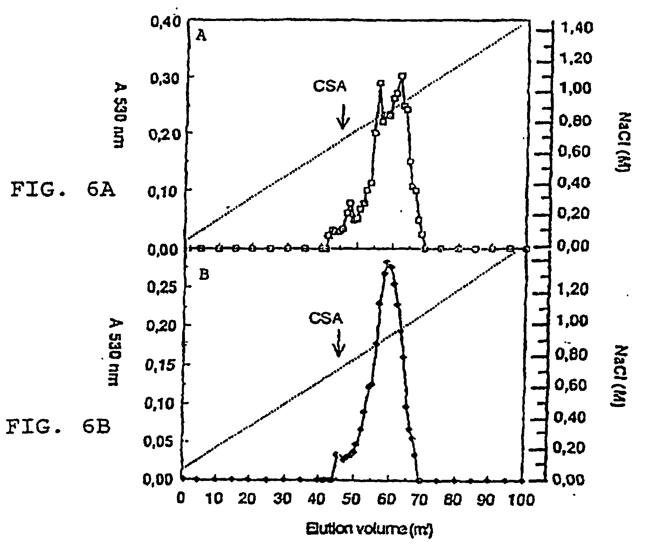

Les figures 6A et 6B sont des profils d'élution sur colonne de DEAE-Trisacryl respectivement des fractions HSM (Fig. 6A) et HSS (Fig. 6B), en présence de fractions chondroïfines sulfates (CSA) pour le calibrage de la colonne.

### EXEMPLE 1: Préparation et sélection des CMDBS

### a) Préparation des CMDBS

Les CMDBS sont des dextranes substitués par des groupements carboxymethyl, benzylamide et benzylamide sulfonate. La méthode de synthèse des CMDBS peut être celle décrite par M.MAUZAC et J.JOSEFONVICZ dans Biomaterials 1984,5,301-304.

Selon ce procédé, le carboxyl méthyle dextrane (CMD) est préparé à partir de dextrane par substitution de quelques unités glycosylées avec des groupes carboxyliques sur le carbone en position 5 ou 6.

Dans une deuxième étape, la benzylamide est couplée aux groupes carboxyliques pour former le carboxyméthyl-benzylamide dextrane (ou CMBD). Enfin quelques noyaux aromatiques du benzylamide sont sulfonés pour aboutir au carboxyméthyle dextrane benzylamide sulfonate ou CMDBS.

Les sels de sodium de ces dérivés sont ultrafiltrés, lyophilisés et dissous dans le tampon approprié avant utilisation.

La formule générale des CMDBS est représentée sur la figure 1.

Les CMDBS possèdent une distribution statistique des différents substituants. Les pourcentages pour chaque type de CMDBS sont déterminés par les méthodes classiques.

### b) Sélection des CMDBS

### i:Tests de protection et de stabilisation des FGFs

Lors de la synthèse des CMDBS il est possible de contrôler le taux de substitution de chacun des groupements par modification des conditions de la réaction de substitution. Le contrôle des paramètres comme la température, le temps de réaction, les concentrations relatives des constituants et le nombre de réaction de substitution etc... permettent d'obtenir un très grand nombre de polymères substitués. La substitution des hydroxyles par le carboxymethyl sur les carbones en position 5 et 6 permet d'obtenir des taux de carboxyméthylation allant de 0 à 200% (100% pour chacun des carbones en position 5 et 6). Le groupement carboxyméthyl peut être à son tour partiellement ou totalement utilisé pour la fixation de la benzylamide. Les groupes benzylamides peuvent être partiellement ou totalement utilisés pour la sulfonation. Les dextranes substitués fonctionnalisés utilisés selon l'invention sont parmi ceux spécialement décrits dans le brevet français n°2.461.724. Outre la capacité à stabiliser et protéger les facteurs de croissance de la famille FGF comme décrit dans la publication de Tardieu et coll J.Cell.Physio.1992 150 p 194 à 203 ; et dans le brevet Français N°2.461.724; le CMDBS sélectionné doit pouvoir interagir avec au moins un membre de la famille des facteurs de croissance de la famille TGF bêta selon une méthode d'évaluation décrite ci-dessous et protéger les TGF bêta contre une protéolyse.

### ii: Evaluation des capacités d'interactions entre CMDBS et les facteurs de croissance de la famille TGF bêta.

Afin de mesurer la capacité de certains CMDBS à interagir avec les membres de la famille TGF bêta et de par cette interaction protéger les TGF bêta, un test de criblage a été établi. Ce test consiste à mesurer la capacité du CMDBS sélectionné à permettre au TGF bêta de garder son activité biologique malgré un traitement protéasique.

Dans l'exemple ci dessous le CMDBS utilisé est le lot 26.2 défini par un taux de substitution de 110% de motifs carboxyméthyles, 3,6% de motifs benzylamides et 36,5% de motifs sulfonates et possède une activité anti coagulante de 4 UI/mg (Unités Internationales). L'activité anti-complément de ce lot est de 1,1 µg de CH50 mesurée selon Mauzac et al. (précédemment cités).

L'héparine utilisée comme témoin provient des établissements Sanofi.(Institut Choay) et présente une activité anticoagulante de 175 UI/mg

Le TFG bêtal est préparé à partir de plaquettes sanguines humaines selon le protocole décrit dans de nombreuses publications et couramment utilisés par l'homme de l'art, par exemple dans la publication Growth Factors and their Receptors 1992 , vol. 1 PP 419-472 par A. Roberts et M.Sporn édité par A. Roberts et M.Sporn et publiée par Springer Verlag Berlin. Le test d'activité biologique du TGF bêta utilisé dans cet exemple est celui de l'inhibition de croissance des cellules CCL64 (provenant de l'American Tissue Culture Collection). Cette inhibition est mesfurée par la capacité du TGF b à inhiber l'incorporation de Thymidine tritiée d'une manière dose dépendante dans ces cellules CCL64 stimulées par le facteur de croissance FGF ou par du sérum de veau foetal selon le protocole décrit par Van Zolen dans Progress in Growth Factor Resarch, 1990 ,2 p. 131 à 152. Le TGF bêta est utilisé à deux doses, l'une correspondant à la capacité d'inhibition de 50% de l'incorporation de Thymidine tritiée (définie comme l'unité d'activité inhibitrice) l'autre, correspondant à la capacité d'inhibition de 100%. Dans cet exemple les valeurs obtenues sont de 250 pg de TGF bêta pour obtenir l'unité d'activité d'inhibition sur les cellules CCL64 cultivées dans 1 ml de milieu de culture. Le 100% d'inhibition est obtenu avec 1ng de TGF bêta dans 1 ml de milieu de culture.

Un échantillon de 50ng de TGF bêta dans du tampon phosphate salin contenant 0.1% de serum albumine bovine (provenant de la société SIGMA à Saint Louis USA) est incubé seul, ou associé soit à 5000 ug de CMDBS, soit à 5000 µg d'héparine, avec ou sans 500 µg de trypsine. Le volume final de la solution incubée est ajusté à 1 ml et l'incubation est effectuée à 37°C durant un temps variable (10 minutes dans l'exemple décrit tableau 1).

Des échantillons d'un volume de 21) µl de chacune des réactions d'incubation sont prélevés et ajoutés aux cellules CCL64 cultivées dans des plateaux de 24 puits contenant chacuns un millilitre de milieu de culture selon le protocole décrit par E.Zohlen mentionné ci dessus. Dans ces conditions la concentration finale de TGF bêta par puits est de 1ng/ml. La tableau 1 résume les résultats obtenus dans diverses conditions et montre l'effet protecteur du CMDBS. Ainsi après 10 mn d'incubation à 37°C, 75% de l'activité biologique du TGF bêta est encore présente, alors que l'héparine qui pourtant peut se fixer au TGF bêta (Mac Caffrey et al., J. of Cell Physiology, 1992, vol.52, 430-440) ne protège pas le TGF bêta contre cette dégradation protéolytique (il reste moins de 20% d'activité biologique). Il est à rappeler que dans le cas des FGFs l'héparine assure une protection contre la protéolyse induite par la trypsine.(Tardieu et al., Journal of Cellular Physiology, 1992, 150: 194-203).

Il a été vérifié que le CMDBS n'avait pas de pouvoir inhibiteur sur l'activité de la trypsine (tableAU 2). Ainsi,10 µg de trypsine ont été incubés soit avec un substrat (S.87 fourni par la société Serbio, Paris et utilisé selon les recommandations de ce fournisseur) ou soit avec ce substrat et un inhibiteur de la trypsine tel celui provenant du soja (comme le Soyabean trypsin inhibitor ou STI de chez Sigma) ces incubations étant faites en l'absence ou en présence de quantités variables de CMDBS (lot AM26). L'activité enzymatique de la trypsine a été mesurée par absorption spectrophotométrique du produit de transformation du S 87 en fonction du temps d'incubation.

### Exemple 2: sélection d'autres HBGFPP :

Une préparation commerciale de protéoglycosaminoglycanne et glycosaminoglycannes, le sulodexide, a été sélectionnée selon sa capacité à interagir avec les facteurs de croissance de la famille des FGF ainsi qu'avec ceux de la famille des TGF bêta.

Des préparations d'héparane sulfate obtenues par fractionnement du mésoglycan et du sulodexide ont d'autre part été testées.

Le mésoglycan et le sulodexide ont été fournis par la Société Sigma Chemical Co , Saint Louis MO USA. Leurs propriétés sont résumées dans le tableau 3.

Les cellules utilisées dans cet exemple sont les cellules CC139 qui proviennent de l'American Tissue Culture Collection. Les conditions de culture et de tests de mesure d'activité biologique des FGFs sont les mêmes que celles décrites dans la publication Tardieu et coll J.Cell.Physiol. 1992. Les facteurs de croissance FGF utilisés sont les formes recombinantes FGF1 et FGF 2.

### a) Effet du sulodexide sur l'activité biologique des FGFs in vitro.

Dans ces expériences le FGF1 ou 2 est utilisé à une dose correspondant à la dose efficace (notée ED50) pour induire une stimulation de l'activité biologique de 50% de la dose induisant la stimulation maximale .L'activité biologique est mesurée par la capacité d'induire une augmentation de l'incorporation de thymidine tritiée dans les cellules selon les protocoles largement décrits dans de nombreuses publications dont celle de Tardieu et coll mentionnée précédemment et également dans le brevet français N°2 644 066.

Dans cet exemple l'ED50 est de 5 ng/ml pour le FGF1 et de 3 ng/ml pour le FGF 2, valeurs mesurées expérimentalement (Figs.2a et 2b). La même expérience de stimulation en fonction de la dose de FGF est effectuée en présence de 10 µg/ml de Sulodexide ou 20 ug/ml d' Héparine. La figure 2 montre que dans ces conditions l'ED50 devient 0.4 ng/ml et 0.2 ng/ml respectivement pour les FGF1 et FGF2 en présence de ces doses de Sulodexide ou d'Héparine. Outre cette capacité à potentialiser l'activité biologique des FGFs les HBGFPP protègent les FGFs contre les dégradations thermiques ainsi que contre l'inactivation induite par 1' action protéolytique de la trypsine.(Figs.4 et 5). De la même manière ces HBGFPP protègent FGF1 et 2 contre une inactivation induite par l'activité protéolytique de la trypsine (Figs.5a et 5b).

### b) Effets protecteurs du sulodexine, du dextrane, du dextrane Sulfate et de la sucrase vis-à-vis des TGFbêta.

Plusieurs autres composés ont été évalués : le dextrane sulfate (Sigma Chemical, de poids moléculaire 40.000, le dextrane ayant servi à la synthèse du CMDBS (également de chez Sigma) de la sucrase ou sucrose octasulfate (fournie par D. Bar Shalom, Société BUKH MEDIC, au Danemark). Certains de ces composés ont été choisis car ils protègent et stabilisent les FGF tels que la sucrase se confère au brevet US N° 5202311 ou le dextrane sulfate se confère au brevet japonais n° 138 907/88). Le dextran est celui qui a servi a la synthèse du CMDBS AM26.

L'expérience de .protection de l'activité biologique des TGFbêta a été réalisée de la même manière qu'avec les CMDBS ainsi que décrit dans l'exemple 1 ii. Le mélange d'incubation contient 50 ng de TGF bêta (dans 0.1 % d'albumine sérique de bovin) et de la trypsine (500 µg). Le Mésoglycanee ou le Sulodexide ou le dextran sulfate ou le dextran ou la sucrase sont utilisés à la dose de 5000 µg.

L'activité biologique du TGFbêta est mesurée comme décrit ci-dessus après une dilution de 50 fois et en utilisant des cellules CCL64.

Les résultats sont présentés dans le tableau 4.

Ces résultats illustrent que, comme certains CMDBS capables de répondre aux deux critères de sélection vis-à-vis des FGF et TGFbêta, le sulodexide présente une activité protectrice significative pour les TGFbêta.

### c) Isolement de la fraction Héparane Sulfate du Sulodexide et du Mésoglycan.

Le Sulodexide et le Mésoglycan correspondent à des mélanges de plusieurs substances dont l'essentiel est constitué de différents glycosaminoglycanes (GAG).

Par une première étape de purification, il a été établi qu'un gramme de produit sec de chacun de ces deux produits contenait respectivement 874 mg pour le mésoglycan et 795 mg pour le sulodexide de GAG totaux.

Cette purification a été obtenue en soumettant ces produits solubilisés à une chromatographie échangeuse d'ions (DEAE - Trisacryl) pour enlever tous les contaminants protéiques. Les GAG totaux ont alors été purifiés en éluant le gel de DEAE avec une solution d'acétate de sodium, pH 4, contenant 1,5 M NaCl.

Après une phase de dialyse extensive contre de l'eau, 60 mg de chaque produit de GAG ont été digérés par la chondroïtinase ABC pendant une nuit à 37°C ( 1 unité par mg de GAG). Cette enzyme dégrade tous les GAG à l'exclusion des héparanes sulfates (HS). Les produits de digestion ont été soumis à une chromatographie sur tamis moléculaire (G50 Sephadex, colonne de 1,8 x 95 cm). L'élution est ensuite réalisée en tampon bicarbonate d'ammonium sous un débit de 18 ml/h. Le matériel non digéré qui correspond à des GAG de nature HS est collecté dans le volume mort d'élution de la colonne.

Les concentrations en GAG sont calculées à partir de leur contenu en acide uronique par la méthode au carbazole (Bitter T. et Muir H.M., 1962, Anal. Biochem 4, 330-334).

Ces dosages ont permis de préciser la composition suivante de chacun des produits:

| | Sulodexide | Mésoglycan |
|---|---|---|
| GAG totaux | 79 % | 87 % |
| Fraction Héparane Sulfate (HS) | 48 % | 52 % |
| Autres GAG | 31 % | 35 % |

Les fractions HS de chacun de ces deux produits ont été chromatographiées à nouveau sur un gel de DEAE Trisacryl. 1 mg de chaque fraction HS, purifiée à partir du mésoglycan (Fig. 6A) ou du sulodexide ( Fig. 6B), dans 3 ml a été déposé sur une colonne équilibrée avec du tampon 0,05 M NaCl, 0,05 M TMS-Hel pH 7,5. Après un lavage de la colonne par 10 volumes du même tampon suivi d'un lavage par 10 volumes d'un tampon 0,05 M NaCl, 0,05 M d'acétate de sodium pH 4, le matériel fixé à la colonne est désorbé par un gradient salin allant de 0,05 M NaCl à 1,5 M NaCl dans le même tampon acétate. 1 ml de chaque fraction collectée a été dosé par la méthode au carbazole.

Le matériel correspondant aux constituants HS de chacun des produits d'origine présente approximativement le même profil d'élution et donc à peu près la même charge apparente. Ce maximum du pic d'élution est obtenu pour une concentration saline de 0,94 M NaCl. Une fraction définie de chondroïtine sulfate (CSA) a été soumise au même protocole en vue de calibrer la chromatographie. Cette fraction CSA qui ne contient qu'un groupe sulfate par disaccharide est élué à la force ionique de 0,72 M NaCl.

Ces résultats montrent que la fraction HS contient plus de groupements sulfates que les CSA de référence. La fraction HS présente environ deux groupes sulfates par unité dissacharidique.

Ces fractions ont été testées pour connaître leur pouvoir protecteur vis-à-vis du TGF *β* et du FGF en comparaison des pouvoirs établis avec les produits bruts respectifs.

### Evaluation semi-quantitative des effets protecteurs du FGF par différents polymères.

Comme décrit ci-dessus, une quantité constante de FGF radioactif est incubée dans des conditions différentes. Après autoradiographie des produits de la réaction, la quantité de FGF radioactif non dégradé est quantifiée par densitométrie. Les valeurs correspondent au pourcentage de FGF radiomarqué retrouvé par rapport à la quantité déposée en début de réaction. (Tableau 5).

Les résultats des tableaux 4 et 5 montrent que les fractions HSM et HSS issues respectivement du mésoglycan et du sulodexide présentent des effets protecteurs supérieurs à ces deux compositions et proches de 100%.

### EXEMPLE 3: Effets inhibiteurs in vitro des CMDBS et des glycosaminoglycanes sur l'activité de l'élastase leucocytaire et sur la plasmine.

Les pouvoirs d'inhibition de différents CMDBS et de leurs composés intermédiaires de leur synthèse, ont été établis pour l'élastase leucocytaire et la plasmine.

L'élastase leucocytaire purifiée a été obtenue par Elastin Products Co (Owenville, MO, USA) et la plasmine chez SIGMA.

L'inhibition des activités enzymatiques par ces différents composés est effectuée à 37°C dans un bain thermostaté. Les enzymes considérées sont mises en solution dans un tampon Tris-HCL 100 mM, pH8 pour l'élastase et pH 7,4 pour la plasmine, en présence de 0,02% d'azide de sodium et de 0,01% Triton X100 pour la plasmine. Les concentrations des substrats et celles des enzymes.sont : 0,10 mM MeO-Suc-Ala-Ala-Pro-Val-pNA (paranitroanilide) pour l'élastase à 8,3 nM et 0,20 mM dVal-Leu-dLys-pNA pour la plasmine à 77 nM. Pour chacune des conditions est établi l'IC50.

Le tableau 6 donne les résultats obtenus dans lesquels, le lot AM6 correspond à un dextrane T40 de 40 000 kD. Le lot EM5 correspond à un dextrane T10 de 10 000 kD. Les produits intermédiaires de synthèse sont répertoriés d'après les sigles désignés ci-dessus indexés d'un numéro qui précise le nombre de chacune des réactions de substitution.

Les valeurs des IC50 démontrent que les CMDBS ont des effets inhibiteurs de type hyperbolique non compétitif sur l'activité de l'élastase leucocytaire comparables à ceux de l'héparine, l'un des meilleurs inhibiteurs de cette activité (Ki de l'ordre de 1 nM). Les CMDBS exercent de plus et à l'inverse de l'héparine des effets inhibiteurs sur la plasmine.

Il ressort en outre du tableau 6 que les effets inhibiteurs des fractions HSM et HSS sont supérieurs à ceux du mésoglycan et du sulodexide, respectivement.

### Exemple 4: Effet cicatrisant du CMDBS sur les anastomoses du colon

Le lot de CMDBS utilisé dans cet exemple est celui décrit dans l'exemple n°1. Une solution de CMDBS est réalisée à 50 µg par ml.dans un sérum physiologique. Des rats Wistar (Wi/Wi , Ico , Iffa CredoFrance) mâles pesant 250 à 300 g sont anesthesiés au penthobarbital sodique . Une laparotomie médiane sous ombilicale est pratiquée sur 1,5 cm.La jonction rectocolique est individualisée puis sectionnée au bistouri. Les deux berges de l'anastomose sont alors imbibées d'une solution contenant soit du CMDBS soit un tampon physiologique et ce durant 2 minutes L'anastomose est réalisée selon un mode termino-terminal en un plan par cinq points séparés de fil de polyglactine 910 de diamètre 6/0.Les noeuds sont faits de 6 boucles. La fermeture pariétale se fait en deux plans. Les rats sont répartis en deux groupes de 7 rats. Les expériences sont faites en double aveugle. Les prélèvements sont effectués au 2ème, 4ème et 7ème jours post opératoire. L'étude de la qualité de la cicatrisation se fait par mesure de la pression de rupture de l'anastomose mesurée en millimètres de mercure .Le colon est sectionné 2 cm de part et d'autre de l'anastomose , une des extrémités est reliée à une seringue autopousseuse qui injecte de l'eau à débit faible et constant, l'autre extrêmité est reliée à un manomètre La pression est enregistrée en permanence sur un rouleau de papier. L'analyse du tracé permet de déterminer, la pression de rupture. Les valeurs obtenues sont présentées dans le tableau 7 ci-après.

Conclusions: Au 2ème jour (48 Heures après l'opération) une différence très nette , de l'ordre de 3 fois supérieure, est mesurée entre les forces de ruptures des anastomoses effectuées avec et sans traitement au CMDBS. Cette moyenne est statistiquement significative (p ≤ 0,05 ). Au 4ème jour cette différence est nettement moins importante et correspond à une résistance augmentée d'environ 10 % en faveur des animaux traités au CMDBS. Au 7 ème jour on n'observe plus de différence avec ou sans traitement. L'intérêt du traitement au CMDBS apparaît évident puisqu'il permet d'assurer une solidité bien accrue de l'anastomose au tout début de la cicatrisation qui est précisément le moment crucial .En effet les risques de rupture de l'anastomose apparaissent toujours dans les tous premiers jours.

### Exemple 5 : Effet cicatrisant du CMDBS dans des colites- induites par l'acide acétique.

Des groupes de 6 rats mâles Sprague Dawley, d'environ 200g ,sont mis en jeune hydrique 24H avant l'essai. Le jour du test, les animaux sont anesthésiés au penthobarbital (30 mg/kg) par voie intraperitonéale. Après laprotomie, une instillation colique de 2ml d'acide acétique à 5% est effectuée à 2cm du caecum. L'acide acétique est laissé en contact 10 secondes puis le côlon est rincé par 3 ml de tampon Phosphate Saline (PBS) à pH 7. La plaie est ensuite refermée. 72 h plus tard, les animaux sont sacrifiés par élongation cervicale. Les altération coliques sont cotées selon le barème suivant:
0: côlon normal
1:congestion
2:nécrose superficielle de la muqueuse
4:nécrose importante de la muqueuse accompagnée d'oedème.
5:perforation

### Traitement:

Un premier groupe contrôle de six rats ne reçoit pas de traitement.

Un deuxième groupe reçoit 50 µg d'une solution aqueuse de CMDBS (lot AM26), administrée par voie orale matin et soir pendant les trois jours.

Un troisième groupe reçoit du Sucralfate (commercialisé sous le nom d'ULCAR laboratoires pharmaceutiques Fabre). La dose utilisée est celle recommandée par ce fourniseur (500 mg/kg, soit 100mg par rat) par voie orale 2 fois par jour pendant 3 jours.

### Analyse Statistique:

Pour chaque lot d'animaux, la moyenne et l'erreur standard ont été calculées. La comparaison statistique est effectuée à l'aide du test non paramétrique de White, par rapport au témoin.

Les résultats sont présentés dans le tableau 8 ci-après.

Comme l'illustrent ces résultats, le CMDBS réduit d'environ 75%,(effet significatif, p<0.05) la gravité des lésions coliques induites par l'acide acétique. En comparaison le sucralfate n'a pas d'effet significatif sur la cicatrisation de ces ulcères.

### Exemple 7.

### Compositions à base de HBGPP.

Dans les exemples 5 et 6 décrits ci-dessus le CMDBS est en solution aqueuse saline tamponnée (tampon PBS) ou sérum physiologique à des concentrations de 50 µg/ml. L'administration est locale ou per os; elle peut être intraveineuse . D'autres compositions peuvent être proposées sous réserve que la dose de CMDBS atteignant le site de la lésion soit comprise entre 1 et 1000 µg.

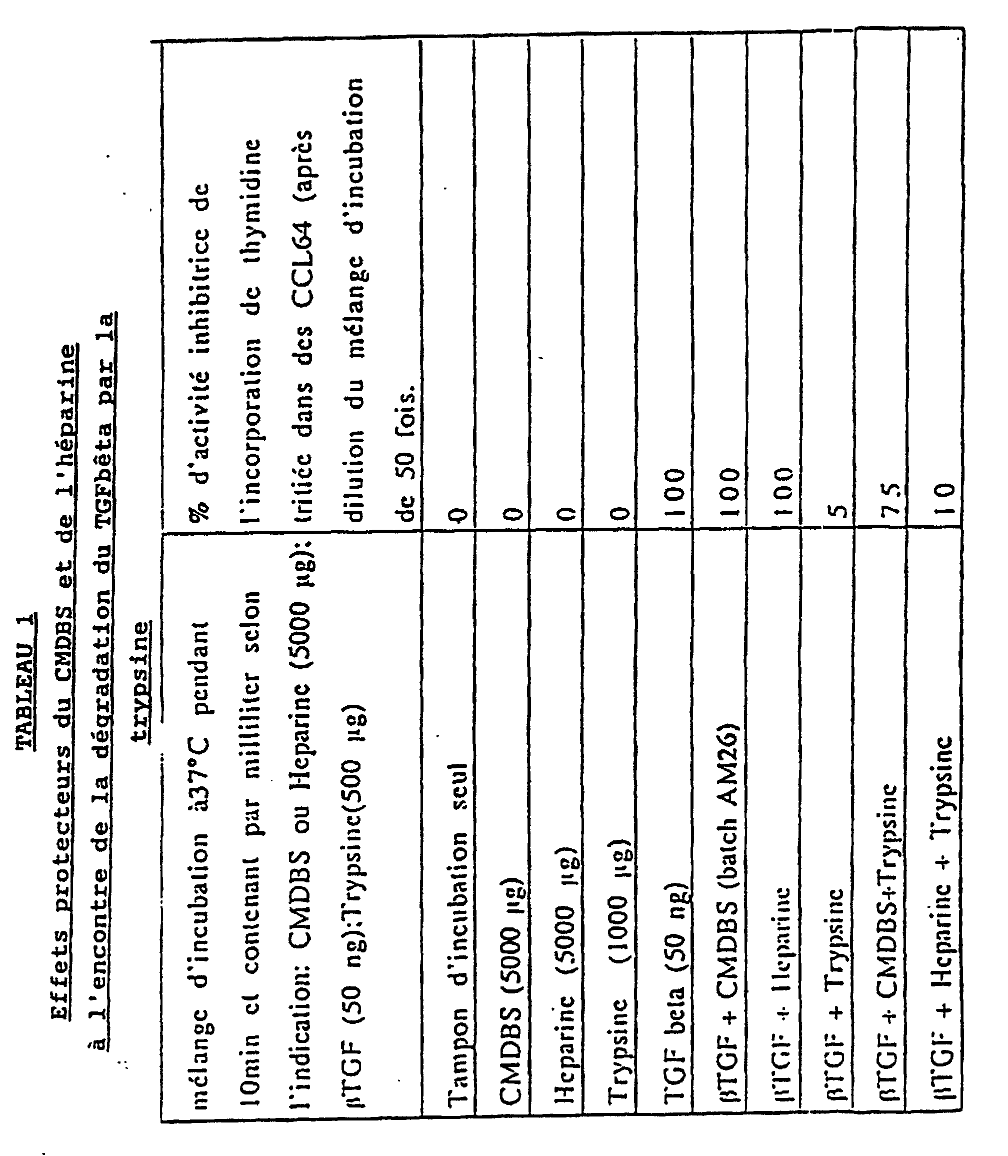

**TABLEAU 2**

| Effet non inhibiteur du CMDBS vis-à-vis de la trypsine | |
|---|---|
| Trypsine (10µg/ml+ S87 | 100 |
| Trypsine+S87+5µg/ml CMDBS | 100 |
| Trypsine+S87+50µg/ml CMDBS | 100 |
| Trypsine+S87+500µg/ml CMDBS | 100 |
| Trypsine+S87+STB1 | 0 |

**TABLEAU 3 :**

| Origine, activité anticoagulante et composition partielle du Mésoglycan et du Sulodexide (informations du fournisseur | | |
|---|---|---|
| | Sulodexide | Mésoglycan |
| Origine | duodénum de porc | aorte |
| Activité anticoagulante | 50-70 IU/mg | < 50 IU /mg |
| Composition chimique | | |
| Dermatane sulfate | 20 - 35 % | 25 - 60 % |
| Chondroitine Sulfate | 2-7% | 3-15% |
| Héparane sulfate | + | + |

**TABLEAU 4**

| Protection du TGFbêta par divers polymères | |
|---|---|
| TGF bêta | 100 % |
| TGF bêta + trypsine | 0% |
| TGF bêta + mésoglycan | 100% |
| TGF bêta + mésoglycan + trypsine | 50 % |
| TGF β+HSM | 100% |
| TGF β + HSM + trypsine | 75% |
| TGF beta + sulodexide | 100 % |
| TGF beta + sulodexide + trypsine | 20% |
| TGF β+HSS | 100% |
| TGP β + HSS + trypsine | 45% |
| TGF bêta + Dextrane | 100% |
| TGF bêta + Dextrane + trypsine | 0% |
| TGF bêta + Dextrane Sulfate | 100% |
| TGF bêta + Dextrane Sulfate + trypsine | 0% |
| TGF bêta + Sucrase | 100% |
| TGF bêta + Sucrase + trypsine | 0% |
| HSM = Héparanes Sulfates purifiés à partir du Mésoglycan MSS = Héparanes Sulfates purifiés à partir de Sulodexide | |

**TABLEAU 5**

| Protection du FGF par divers polymères | |
|---|---|
| | PROTECTION EN % |
| FGF seul | 100 |
| FGF + Trypsine | 0 |
| FGF + Trypsine + Héparine | 100 |
| FGF + Trypsine + Mesoglycan | 75 |
| FGF + Trypsine + Sulodexide | 70 |
| FGF + Trypsine + Mesoglycan traité | 0 |
| Heparinase | |
| FGF + Trypsine + Sulodexide traité | 0 |
| Héparinase | |
| FGF + Trypsine + Héparine Traité | 0 |
| Héparinase | |
| FGF + HSM + Trypsine | 95 |
| FGF + HSS + Trypsine | 90 |

**TABLEAU 6**

| Inhibition des activités de l'élastase et de la plasmine | | |
|---|---|---|
| Composés testés | Elastase Leucocytaire | Plasmine |
| | IC₅₀ en µg/ml | IC₅₀ en µg/ml |
| CMDBS lot AM6 | 2,2 | 1,5 |
| T40 | > 100 | > 100 |
| CMDBS lot AM5 | 10 | 7 |
| T10 CMD2B | 50 | 53 |
| T10 5CMD1B | > 100 | > 100 |
| T10 3CMD | > 100 | > 100 |
| T10 | > 100 | > 100 |
| Mesoglycan | 72 | 65 |
| HS Mesoglycan | 20 | 22 |
| Sulodexide | 79 | 75 |
| HS Sulodexide | 25 | 20 |
| Héparine | 1,8 | |
| Lipo-héparine | | 0,5 |
| HSM = Héparanes Sulfates purifiés à partir du Mésoglycan MSS = Héparanes Sulfates purifiés à partir de Sulodexide | | |

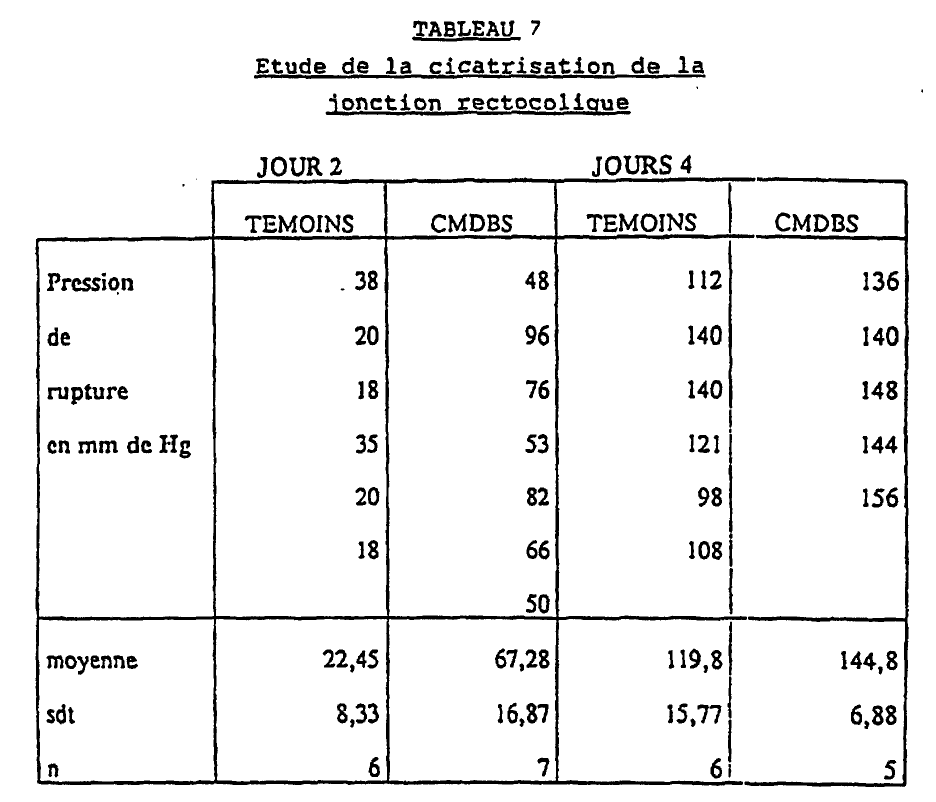

**TABLEAU 8**

| Effet du CMDBS sur des colites induites par l'acide acétique | |
|---|---|
| Traitement | Scores d'altération |
| Témoins | 2.83 ± 0.48 |
| CMDBS 50µg/rat per os | 0.70 ± 0.37 |
| Sucralfate 100 mg/rat per os | 2.25 + 0.48 |

## Revendications

1. Utilisation d'au moins un polymère ou un biopolymère, appelés HBGFPP à l'exclusion du mésoglycan, protégeant spécifiquement les facteurs de croissance des familles des FGF et TGF bêta de la dégradation trypsique et n'inhibant pas de manière significative la coagulation, pour la fabrication d'un médicament pour le traitement des lésions du tractus digestif et des tissus dérivés primaires ou secondaires de l'endoderme et du mésoderme.

2. Utilisation selon la revendication 1, **caractérisée en ce que** le polymère ou biopolymère présente une activité anti-coagulante inférieure à 50 unités internationales par mg de polymère.

3. Utilisation selon l'une des revendications 1 et 2 , **caractérisée en ce que** ledit polymère n'active substantiellement pas le système du complément.

4. Utilisation selon l'une des revendications 1 à 3 **caractérisée en ce que** ledit polymère potentialise in vitro les FGF.

5. Utilisation selon l'une des revendications 1 à 4, **caractérisée en ce que** ledit polymère inhibe substantiellement les activités protéasiques de l'élastase et/ou de la plasmine.

6. Utilisation selon l'une des revendications 1 à 5, **caractérisée en ce que** ledit polymère ou biopolymère est un polysaccharide.

7. Utilisation selon la revendication 6, **caractérisée en ce que** ledit polysaccharide est principalement composé de résidus glucose.

8. Utilisation selon la revendication 6, **caractérisée en ce que** le polysaccharide comprend des résidus giucosamine et/ou d'aciade uronique.

9. Utilisation selon la revendication 8, **caractérisée en ce que** le polysaccharide comprend des dimères glucosamine-acide uronique.

10. Utilisation selon l'une des revendications 8 et 9, **caractérisée en ce que** ledit polysaccharide est un glycosaminoglycane, ou un sulfate d'un de ces composés, éventuellement associé à un lipide, un peptide ou un protide.

11. Utilisation selon l'une des revendications 1 à 6, **caractérisée en ce que** ledit polysaccharide est un dextrane substitué.

12. Utilisation selon la revendication 11, **caractérisée en ce que** ledit polysaccharide est un dextrane substitué par des résidu carboxyméthyle, benzylamide et benzylamine sulfonate (CMDBS).

13. Utilisation selon l'une des revendications 1 à 5, **caractérisée en ce que** ledit polymère est de nature non-osidique.

## Patentansprüche

1. Verwendung wenigstens eines Polymers oder Biopolymers mit der Bezeichnung HBGFPP mit Ausnahme von Mesoglycan, das insbesondere die Wachstumsfaktoren von FGF- und TGF-β-Familien vor dem Trypsinabbau schützt und die Koagulation nicht signifikant hemmt, zur Herstellung eines Medikaments zur Behandlung von Läsionen des Verdauungstrakts und von primären oder sekundären, vom Endoderm und vom Mesoderm abstammenden Geweben.

2. Verwendung nach Anspruch 1, **dadurch gekennzeichnet, dass** das Polymer oder Biopolymer eine antikoagulierende Wirkung von weniger als 50 internationalen Einheiten pro mg Polymer aufweist.

3. Verwendung nach einem der Ansprüche 1 bis 2, **dadurch gekennzeichnet, dass** das Polymer das Komplementsystem im wesentlichen nicht aktiviert.

4. Verwendung nach einem der Ansprüche 1 bis 3, **dadurch gekennzeichnet, dass** das Polymer die FGF in vitro potenziert.

5. Verwendung nach einem der Ansprüche 1 bis 4, **dadurch gekennzeichnet, dass** das Polymer die Proteasenaktivitäten von Elastase und/oder Plasmin im wesentlichen hemmt.

6. Verwendung nach einem der Ansprüche 1 bis 5, **dadurch gekennzeichnet, dass** das Polymer oder Biopolymer ein Polysaccharid ist.

7. Verwendung nach Anspruch 6, **dadurch gekennzeichnet, dass** das Polysaccharid hauptsächlich aus Glucoseresten besteht.

8. Verwendung nach Anspruch 6, **dadurch gekennzeichnet, dass** das Polysaccharid Glucosamin- und/oder Uronsäurereste umfasst.

9. Verwendung nach Anspruch 8, **dadurch gekennzeichnet, dass** das Polysaccharid Glucosamin-Uronsäure-Dimere umfasst.

10. Verwendung nach einem der Ansprüche 8 und 9, **dadurch gekennzeichnet, dass** das Polysaccharid ein Glycosaminoglycan oder ein Sulfat einer dieser Verbindungen ist, das gegebenenfalls mit einem Lipid, Peptid oder Protid verbunden ist.

11. Verwendung nach einem der Ansprüche 1 bis 6, **dadurch gekennzeichnet, dass** das Polysaccharid ein substituiertes Dextran ist.

12. Verwendung nach Anspruch 11, **dadurch gekennzeichnet, dass** das Polysaccharid ein Dextran ist, das mit Carboxymethylen-, Benzylamid- und Benzylaminsulfonat- (CMDBS-)Resten substituiert ist.

13. Verwendung nach einem der Ansprüche 1 bis 5, **dadurch gekennzeichnet, dass** das Polymer von Nicht-Glycosid-Beschaffenheit ist.

## Claims

1. Use of at least one polymer or one biopolymer, called HBGFPP, with the exception of mesoglycan, specifically protecting the growth factors of families of FGFs and beta TGFs from tryptic degradation and not significantly inhibiting coagulation, in the manufacture of a drug for the treatment of lesions of the digestive tract and of primary or secondary derived tissues of the endoderm and the mesoderm.

2. Use according to claim 1, **characterized in that** the polymer or biopolymer presents an anticoagulant activity of less than 50 international units per mg of polymer.

3. Use according to one of claims 1 and 2, **characterized in that** the said polymer does not substantially activate the complement system.

4. Use according to one of claims 1 to 3, **characterized in that** the said polymer potentializes in vitro the FGF.

5. Use according to one of claims 1 to 4, **characterized in that** the said polymer substantially inhibits the proteasic activities of elastase and/or plasmin.

6. Use according to one of claims 1 to 5, **characterized in that** the said polymer or biopolymer is a polysaccharide.

7. Use according to claim 6, **characterized in that** the said polysaccharide is primarily composed of glucose residues.

8. Use according to claim 6, **characterized in that** the polysaccharide comprises glucosamine and/or uronic acid residues.

9. Use according to claim 8, **characterized in that** the polysaccharide comprises glucosamine-uronic acid dimers.

10. Use according to one of claims 8 and 9, **characterized in that** the said polysaccharide is a glycosaminoglycan, or a sulfate of one of these compounds, possibly associated with a lipid, a peptide or a protide.

11. Use according to one of claims 1 to 6, **characterized in that** the said polysaccharide is a substituted dextran.

12. Use according to claim 11, **characterized in that** the said polysaccharide is a dextran substitued with carboxymethyl, benzylamide and benzylamine sulfonate residues (CMDBS).

13. Use according to one of claims 1 to 5, **characterized in that** the said polymer is of non-osidic nature.
